# EUROPÄISCHE PATENTSCHRIFT

(11) **EP 3 008 198 B1**
(45) Veröffentlichungstag und Bekanntmachung des Hinweises auf die Patenterteilung: **10.05.2017**
(21) Anmeldenummer: 14732499.0
(22) Anmeldetag: 17.05.2014
(51) Int. Cl.: C12P 7/26, C12P 13/00, C12N 9/10

(54) **VERFAHREN ZUR HERSTELLUNG VON CATHIN**
PROCESS FOR PRODUCING CATHINE
PROCÉDÉ DE PRODUCTION DE CATHINE

(30) Priorität: 10.06.2013 DE 102013009631
(43) Veröffentlichungstag der Anmeldung: 20.04.2016
(73) Patentinhaber: Forschungszentrum Jülich GmbH, 52425 Jülich (DE)
(72) Erfinder: ROTHER, Dörte, 50354 Hürth (DE); POHL, Martina, 52066 Aachen (DE); SEHL, Torsten, 77654 Offenburg (DE); BARAIBAR, Álvaro Gómez, 44789 Bochum (DE)
(86) Internationale Anmeldenummer: PCT/DE2014/000256
(87) Internationale Veröffentlichungsnummer: WO 2014/198247

(56) Entgegenhaltungen:
- WO-A2-02/02753
- DE-A1- 19 736 104
- TORSTEN SEHL ET AL: "Zwei Schritte in einem Reaktionsgefäß: Enzymkaskaden zur selektiven Synthese von Nor(pseudo)ephedrin aus kostengünstigen Ausgangsmaterialien", ANGEWANDTE CHEMIE (INTERNATIONAL ED. IN ENGLISH), Bd. 125, Nr. 26, 9. Mai 2013 (2013-05-09), Seiten 6904-6908, XP055134536, ISSN: 0044-8249, DOI: 10.1002/ange.201300718
- D. ROTHER ET AL: "S-Selective Mixed Carboligation by Structure-Based Design of the Pyruvate Decarboxylase from Acetobacter pasteurianus", CHEMCATCHEM, Bd. 3, Nr. 10, 31. August 2011 (2011-08-31), Seiten 1587-1596, XP055134569, ISSN: 1867-3880, DOI: 10.1002/cctc.201100054
- TINA GERHARDS ET AL: "Influence of Organic Solvents on Enzymatic Asymmetric Carboligations", ADVANCED SYNTHESIS & CATALYSIS, Bd. 354, Nr. 14-15, 4. Oktober 2012 (2012-10-04), Seiten 2805-2820, XP055134571, ISSN: 1615-4150, DOI: 10.1002/adsc.201200284
- TORSTEN SEHL ET AL: "Efficient 2-step biocatalytic strategies for the synthesis of all nor(pseudo)ephedrine isomers", GREEN CHEMISTRY, Bd. 16, Nr. 6, 1. Januar 2014 (2014-01-01) , Seite 3341, XP055124877, ISSN: 1463-9262, DOI: 10.1039/c4gc00100a
- "Ligation", Wikipedia , 15 April 2016 (2016-04-15), Retrieved from the Internet: URL:https://de.wikipedia.org/wiki/Ligation [retrieved on 2016-04-15]

## Beschreibung

Die Erfindung betrifft ein Verfahren zur Herstellung von Cathin.

Cathin ((1S,2S)-Norpseudoephedrin) kann aus den Blättern des Kathstrauches gewonnen werden und kommt häufig als Appetitzügler zum Einsatz. Es findet auf Grund der sympatomimetrischen, das vegetative Nervenystem anregenden, Funktion Anwendung als Herzkreislaufmittel.

Für die synthetische Herstellung sind nach dem Stand der Technik verschiedene Verfahren bekannt.

So ist in der Veröffentlichung "Stereoselective Synthesis of Norephedrine and Norpseudoephedrine by Using Asymmetric Transfer Hydrogenation Accompanied by Dynamic Kintic Resolution" von Hyeon-Kyu Lee et al. im Journal of Organic Chemistry 2012, S 5454-5460 eine 7-Schrittsynthese bekannt.

Die Veröffentlichung "Efficient Synthesis of Ephedra Alkaloid Analogous Using an Enantiomerically Pure N-[(R)-(+)-α-Methylbenzyl]aziridine-2-carboxaldehyde" von Gwon-II Hwang et al. aus dem J.Org.Chem. 1996, 61, 6183-6188 offenbart ein Verfahren zur Herstellung von Cathin mit hohen Enantiomerenüberschüssen, das von enantiomerenreinem Substrat ausgeht und sehr teuer ist.

Andere Verfahren, wie sie beispielsweise in der Veröffentlichung "Asymetric N1 Unit Transfer to Olefins with a Chiral Nitridomanganese Complex: Novel Stereoselective Pathways to Aziridines or Oxazolines" von Masaaki Nishimura et al. beschrieben werden, offenbaren Verfahren, bei denen Cathin als Nebenprodukt gebildet wird.

T. Sehl et al, Angewandte Chemie, 2013, 125, 6904-6908, beschreibt Enzymkaskaden zur selektiven Synthese von Nor(pseudo)ephedrin aus Benzaldehyd und Pyruvat.

D. Rother, ChemCatChem 2011, 3, 1587-1596, lehrt die Umsetzung von Benzaldehyd und Acetaldehyd zu S-Phenylacetcarbinol in Gegenwart einer Transaminase aus Acetobacter pasteurianus.

Es ist daher die Aufgabe der Erfindung ein einfaches Herstellungsverfahren von Cathin zur Verfügung zu stellen, welches die Nachteile des Standes der Technik überwindet und das mit wenigen Reaktionsschritten auskommt, preisgünstig ist, zu einer hohen Enantiomeren- und Diastereomerenreinheit sowie zu einer hohen Ausbeute führt und möglichst wenige Aufarbeitungsschritte erfordert. Das Verfahren soll einfach in einen größeren Maßstab zu überführen sein.

Ausgehend vom Oberbegriff des Anspruchs 1 wird die Aufgabe erfindungsgemäß gelöst mit den im kennzeichnenden Teil des Anspruchs 1 angegebenen Merkmalen.

Mit dem erfindungsgemäßen Verfahren kann Cathin mit einer Enantiomerenreinheit von > 99 % und Diastereomerenreinheit von 70 %, in einer vorteilhaften Ausgestaltung bis zu 97 %, hergestellt werden. Es kann ein Umsatz von 90 % erzielt werden. Dabei können kommerziell erhältliche Edukte zum Einsatz kommen. Das Verfahren erfordert wenige Aufarbeitungsschritte, ist einfach hoch zu skalieren und kann als Eintopfreaktion durchgeführt werden.

Vorteilhafte Weiterbildungen der Erfindung sind in den Unteransprüchen angegeben.

Im Folgenden soll die Erfindung in ihrer allgemeinen Form beschrieben werden.

Ein (*S*)-selektives Enzym im Sinne der Erfindung ist ein Enzym, welches zu einem (*S*)-konfigurierten Produkt führt.

In einem ersten Reaktionsschritt wird Benzaldehyd mit einem Acetyldonor nach Formel (1) 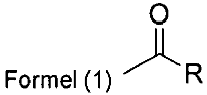 mittels der (*S*)-selektiven Lyase ApPDC-E469G aus Acetobacter pasteurianus zu einem Enantiomerengemisch der Verbindungen nach den Formel (2) und (3) 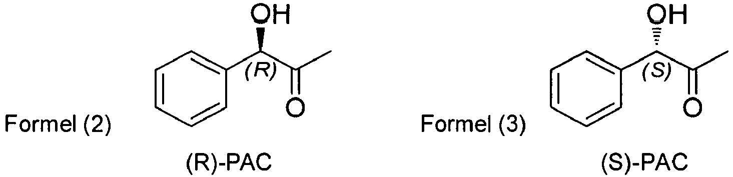 *in vitro* umgesetzt, wobei die Abkürzung PAC Phenylacetylcarbinol bedeutet.

Bei dem Acetyldonor nach Formel (1) kann es sich dabei um Acetaldehyd mit R = H oder Pyruvat mit R = COOH handeln.

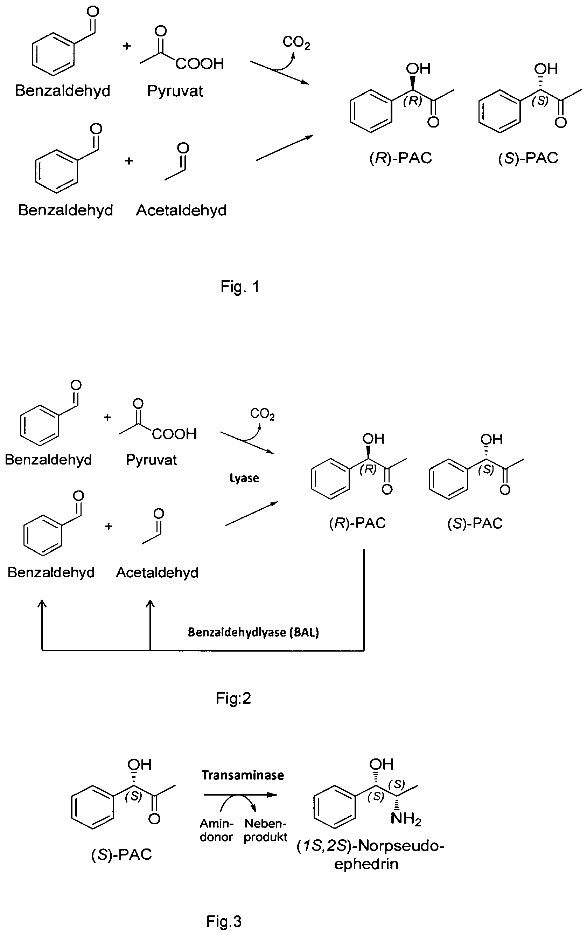

Der Reaktionsweg ist in Figur 1 beispielhaft abgebildet.

Vorzugsweise werden gereinigte und lyophilisierte Enzyme eingesetzt. Die Lyophilisierung hat den Vorteil, dass die Enzyme stabil sind und hohe Enzymkonzentrationen eingesetzt werden können, zudem führen gereinigte Enzyme zu deutlich höheren optischen Reinheiten, also höherem Enantiomeren- und Diastereomerenüberschuß des Produktes.

Weiterhin können Kofaktoren für die (*S*)-selektive Lyase eingesetzt werden, die den Umsatz steigern.

Beispielsweise können als Kofaktoren Magnesiumionen, z.B. als Magnesiumsulfat oder Magnesiumchlorid, mit Thiamindiphosphat eingesetzt werden.

Bei Verwendung von Magnesiumsulfat und Thiamindiphosphat als Kofaktoren ist ein Konzentrationsbereich von 1-5 mM, vorzugsweise 2,5 mM für Magnesiumsulfat und von 5-300 µM, vorzugsweise 100 µM für Thiamindiphosphat bevorzugt.

Die Reaktion kann bei einem pH-Wert von 5 bis 8, vorzugsweise von 6 bis 7,5 durchgeführt werden.

Dafür kann beispielsweise ein Kaliumphosphat-Puffer eingesetzt werden. Es kommen jedoch auch Puffer, wie HEPES, MOPS, TEA oder TRIS-HCl in Betracht.

Der bevorzugte Temperaturbereich liegt bei Raumtemperatur, jedoch kann die Reaktion gut zwischen 20°C und 30°C, insbesondere 25°C durchgeführt werden.

Die Reaktion wird vorzugsweise bei Atmosphärendruck durchgeführt.

Alternativ kann die enzymatische Umsetzung *in vivo* erfolgen. Das hat den Vorteil, dass das Enzym als Kathalysator kostengünstig herzustellen ist.

Dazu können *E.coli* Bakterien als Produktionsorganismen eingesetzt werden.

Dabei können Gene, die für eine Lyase beim ersten Schritt kodieren in einen Vektor ligiert werden.

Vorzugsweise sind die *E.coli* Stämme rekombinant und enthalten Plasmide, die Gene für die (*S*)-selektive Lyase tragen.

Vorzugsweise können die Plasmide die Gene für die o.g. Lyasen beinhalten.

Als Plasmide können beispielsweise pET22b oder pKK233- Grundkörper eingesetzt werden, die die entsprechenden Lyasegene beinhalten.

Die Produktionsorganismen sekretieren das gewünschte Produkt nach Formel (2) und (3) in die wässrige Lösung.

In einer bevorzugten Ausführungsform des erfindungsgemäßen Verfahrens wird die unerwünschte Verbindung nach Formel (2) 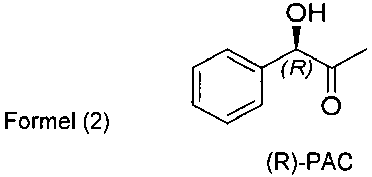 mittels einer Benzaldehydlyase in Benzaldehyd und den Acetaldehyd umgesetzt.

Das Reaktionsschema ist in Figur 2 dargestellt.

Das hat den Vorteil, dass das nicht gewünschte Nebenprodukt nach Formel (2) gespalten wird und das entstehende Benzaldehyd und Acetaldehyd in den Prozess zurückgeführt wird und sich die Ausbeute an dem gewünschten Produkt nach Formel (3) erhöht. Der Enantiomerenüberschuss an (S)-PAC nach Formel 3 kann auf insgesamt > 97 % gesteigert werden. Die Ausbeute in diesem Schritt bezogen auf die Gesamtmenge eingesetztem PAC beträgt 95 %.

Bei der Umsetzung der Verbindung nach Formel (2) ist es vorteilhaft, den Acetyldonor im Reaktionssprozess in einem Überschuss zuzugeben, so dass für die weitere Umsetzung zur gewünschten Zwischenverbindung nach Formel (3) genügend Acetyldonor zur Verfügung steht. Dies ist insbesondere bei flüchtigen Acetyldonoren, wie beispielsweise Acetaldehyd vorteilhaft. Beispielsweise kann gegenüber dem Benzylaldehyd ein 10-facher Überschuss an Acetyldonor eingesetzt werden.

Das Zwischenprodukt wird vorzugsweise abgetrennt. Hierzu kann eine Säulenchromatographie mit Silikagel unter Verwendung von Petrolether : Ethylacetat (90:10) als Trennflüssigkeit eingesetzt werden.

Die so erhaltene Verbindung nach Formel (3) wird in einem zweiten Reaktionsschritt mit einem Amindonor mittels einer (*S*)-selektiven Transaminase zum Endprodukt Cathin ((*1S*, *2S*,)- Norspeudoephedrin) umgesetzt. Die Reaktion ist in Figur 3 schematisch dargestellt.

Für den zweiten Reaktionsschritt, der chemischen reduktiven Aminierung, können Transaminasen eingesetzt werden, die das Substrat umsetzen und (*S*)-selektiv sind. Die Transaminase kann aus einem *Chromobakterium,* beispielsweise *Chromobakterium violacaeum,* vorzugsweise CV2025 sein. Weiterhin können Transaminasen aus *Alcaligens denitrificans, Arthrobacter citreus, Bacillus megaterium, Pseudomonas fluoreszens, Vibrio flurialis* oder *Caulobacter crescentus* eingesetzt werden.

Vorzugsweise werden gereinigte und lyophilisierte Enzyme eingesetzt. Die Lyophilisierung hat den Vorteil, dass die Enzyme stabil sind und hohe Enzymkonzentrationen eingesetzt werden können, zudem führen gereinigte Enzyme zu deutlich höheren optischen Reinheiten, also höherem Enantiomeren- und Diastereomerenüberschuß des Produktes.

Als Amindonoren können beispielsweise (*S*)-alpha-Methylbenzylamin, Benzylamin, Isopropylamin, L-Alanin, (±)-1 -Methyl-3-phenylpropylamin, (±)-1-Aminoindan eingesetzt werden.

Als Kofaktor kann Pyridoxal-5'-phosphat eingesetzt werden.

Vorzugsweise ist die Pyridoxal-5'-phosphat-Konzentration zwischen 100-200 µM.

Die Reaktion kann in einem wässrigen Medium in einem pH-Bereich von 6 bis 11, vorzugsweise pH 7,5 bis 8,5 durchgeführt werden.

Dazu können geeignete Puffer, wie HEPES, Kaliumphosphat, MOPS, TEA oder TRIS-HCl eingesetzt werden.

Der bevorzugte Temperaturbereich liegt bei 25°C, die Reaktion kann jedoch in einem Bereich von 20°C - 30°C gut durchgeführt werden.

Vorzugsweise wird die Reaktion bei Atmosphärendruck durchgeführt.

Alternativ kann die enzymatische Umsetzung mit der (*S*)-selektiven Transaminase *in vivo* erfolgen.

Dazu können *E.coli* Bakterien als Produktionsorganismen eingesetzt werden.

Dabei können Gene, die für eine Transaminase kodieren, in einen Vektor ligiert werden.

Vorzugsweise sind die *E.coli* Stämme rekombinant und enthalten Plasmide, die Gene für eine (*S*)-selektive Transaminase tragen.

Vorzugsweise können die Plasmide die Gene für die o.g. Transaminasen beinhalten.

Als Plasmide können beispielsweise pET29a oder pKK233- Grundkörper eingesetzt werden, die die entsprechenden Transaminasegene beinhalten.

Die Produktionsorganismen sekretieren das gewünschte Produkt nach Formel (3) in die wässrige Lösung.

Die Umsetzungen *in vivo* können bei dem erfindungsgemäßen Verfahren entweder nur für den ersten oder den zweiten Reaktionsschritt oder für beide Reaktionsschritte 1 und 2 durchgeführt werden. Die restlichen Bedingungen können für jeden Schritt genauso gewählt werden, wie für die enzymchemische Umsetzung *in vitro.*

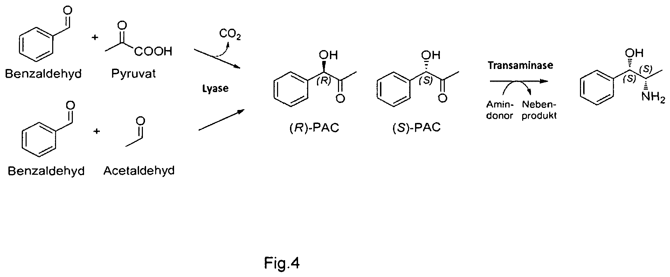

Figur 4 zeigt eine Zusammenfassung der Schritte aus den Figuren 1 und 3.

### Beispiel:

Die 2-Schritt Synthese wurde mit der Kombination der (*S*)-selektiven Lyase ApPDC-E469G aus dem Organismus *Acetobacter pasteurianus* im ersten Schritt und der (*S*)-selektiven Transaminsase CV2025 aus dem Organismus *Chromobacterium violacaeum* im zweiten Schritt erfolgreich durchgeführt. Im ersten Schritt wird Acetaldehyd direkt mit Benzaldehyd bzw. Pyruvat nach Decarboxylierung zu Acetaldehyd mit Benzaldehyd, zum (*S*)-PAC mit einer Enantiomerenreinheit von ca. 70 % verknüpft. Die Ausbeute nach Aufarbeitung betrug 95 %, der *ee* ca. 70 %. Der Umsatz im zweiten Reaktionschritt lag bei 95 %. Insgesamt konnte so über beide Reaktionsschritte ein Gesamtumsatz zu Cathin von ca. 90 % mit einer optischen Reinheit von *ee* > 99 % und de ca. 70 % erzeugt werden.

### Reaktionsbedingungen:

### Schritt 1 (Lyase-Reaktion, Fig.1):

40mM Benzaldehyd, 400 mM Pyruvat, 2,5 mM Magnesiumsulfat, 100 µm Thiamindiphosphat, 0,5 mg/mL ApPDC-E469G (gereinigtes lyophilisirtes Enzym), 100 mM Kaliumphosphat-Puffer, 25°C, Reaktionszeit 48h. Anschließend erfolgte eine Extraktion und eine säulenchromatographische Reinigung des Produkts ((*S*)-PAC). Die Ausbeute betrug 95 %. Alternativ kann die Reaktion auch mit Acetaldehyd anstelle von Pyruvat und/oder mit ganzen Zellen in denen das Enzym ApPDC-E469G überexprimiert vorliegt, anstelle von gereinigtem Enzym durchgeführt werden.

### Schritt 2 (Transaminasereaktion, Figur 3)

10mM (*S*)-PAC, 10mM (*S*)-alpha-Methylbenzylamin, 1 mg/ml gereinigtes Enzym, 25°C, 100 mM HEPES mit 0,1mM Pyridoxal-5'-Phposphat, pH 7,5, Reaktionszeit 24 h. Der Umsatz betrug 95 %. Die Reaktion kann alternativ auch mit lyophlisierten ganzen Zellen, in denen die (*S*)-selektive Transaminase überexprimiert vorliegt, katalysiert werden.

Durch einen zusätzlichen Schritt nach der Lyase-Reaktion kann die Enantiomerenreinheit des Intermediats ((*S*)-PAC) optional erhöht werden. Zum gereinigten (*S*)-PAC (*ee* ca. 70%) werden Pyruvat, ApPDC-E469G und Benzaldehydlyase aus dem Organismus *Pseudomonas fluorescens* zugegeben. Die Benzaldehydlyase katalysiert die selektive Spaltungsreaktion von (*R*)-PAC zu Benzaldehyd und Acetaldehyd. Der entstehende Benzaldehyd dient analog Reaktionsschritt 1 als Substrat für die sukzessiv von dem Enzym ApPDC-E469G katalysierte Carboligationsreaktion von Benzaldehyd und Pyruvat zu (*S*)-PAC. Der ee des Produkts kann so auf *ee* > 97 % (S)PAC erhöht werden, wenn sowohl die Benzaldehydlyase als auch ApPDC-E469G im Reaktionsansatz vorhanden sind. Die Ausbeute, bezogen auf die Gesamtmenge eingesetztem PAC in diesem Schritt, betägt > 91 %.

### Erhöhung der Enantiomerenreinheit von (S)-PAC in Figur 2:

a) 11,2 mM (S)-PAC (*ee* ca. 70%), 200 mM Pyruvat, 2,1 mg/mL ApPDC-E469G, 2 mg/mL BAL (Benzaldehydlyase), 2,5 mM Magnesiumsulfat, 100 µM Thiamindiphosphat , 100µM Kaliumphosphat-Puffer, 25 °C, Reaktionszeit: 48h. Ausbeute: 91,6 %, *ee* ((*S*)-PAC) = 97,4 %.
b) 20,7 mM (*S*)-PAC (*ee* ca. 70 %), 300 mM Pyruvat, 2,1 mg/mL ApPDC-E469G, 2 mg/mL BAL (Benzaldehydlyase), 2,5 mM Magnesiumsulfat, 100 µM Thiamindiphosphat , 100µM Kaliumphosphat-Puffer, 25 °C, Reaktionszeit 48 h. Ausbeute 93,7 %, *ee* ((*S*)-PAC) = 96,7 %.

## Patentansprüche

1. Verfahren zur Herstellung von (1S,2S)-Norpseudoephedrin **dadurch gekennzeichnet,**
**dass** Benzaldehyd in einem ersten Reaktionsschritt mit einem Acetyldonor nach Formel (1) 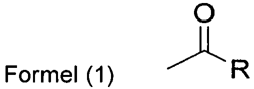 mit R = H oder COOH mittels der (S)-selektiven Lyase ApPDC-E469G aus *Acetobacter pasteurianus* zu einem Enantiomerengemisch nach den Formeln (2) und (3) 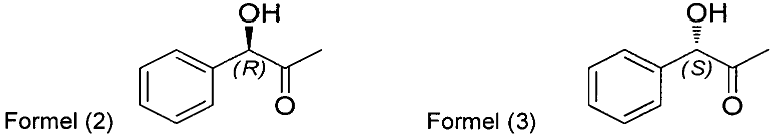 umgesetzt wird und die Verbindung nach Formel (3) in einem zweiten Schritt mit einem Amindonor mittels einer (*S*)-selektiven Transaminase zu (*1S,2S*)-Norpseudoephedrin umgesetzt wird.

2. Verfahren nach Anspruch 1,
**dadurch gekennzeichnet,**
**dass** als Kofaktor ein Magnesiumsalz sowie Thiamindiphosphat eingesetzt wird.

3. Verfahren nach einem der Ansprüche 1 oder 2,
**dadurch gekennzeichnet,**
**dass** Schritt 1 bei einem pH-Wert von 5 bis 9 durchgeführt wird.

4. Verfahren nach einem der Ansprüche 1 bis 3,
**dadurch gekennzeichnet, dass** die Verbindung nach Formel (2) 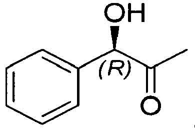 als Produkt des ersten Reaktionsschrittes mittels einer Benzaldehydlyase in Benzaldehyd und Acetaldehyd, umgesetzt wird.

5. Verfahren nach einem der Ansprüche 1 bis 4,
dadurch gekenzeichnet,
dass der Acetyldonor in einem Überschuss zugegeben wird.

6. Verfahren nach einem der Ansprüche 1 bis 5,
**dadurch gekennzeichnet, dass** das Zwischenprodukt nach Formel (3) 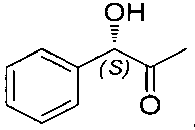 abgetrennt wird.

7. Verfahren nach einem der Ansprüche 1 bis 6, **dadurch gekennzeichnet, dass** die Verbindung nach Formel (3) 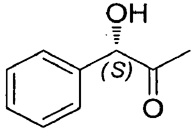 im zweiten Schritt mit einer (*S*)-selektiven Transaminase aus einem *Chromobakterium, Alcaligens denitrificans, Arthrobacter citreus, Bacillus megaterium, Pseudomonas fluoreszens, Vibrio flurialis* oder *Caulobacter crescentus* umsgesetzt wird.

8. Verfahren nach Anspruch 7,
**dadurch gekennzeichnet, dass** als (*S*)-selektive Transaminase CV2025 aus *Chromobakterium violacaeum* eingesetzt wird.

9. Verfahren nach einem der Ansprüche 1 bis 8,
**dadurch gekennzeichnet,**
**dass** im zweiten Schritt als Amindonor eine Komponente aus der Gruppe Methylbenzylamin, Benzylamin, Isopropylamin, L-Alanin, (±)-1-Methyl-3phenylpropylamin oder (±)-1- Aminoindan eingesetzt wird.

10. Verfahren nach einem der Ansprüche 1 bis 9,
**dadurch gekennzeichnet,**
**dass** bei Schritt 2 Pyridoxal-5'-phosphat als Kofaktor eingesetzt wird.

11. Verfahren nach einem der Ansprüche 1 bis 10,
**dadurch gekennzeichnet,**
**dass** Schritt 2 bei einem pH-Wert von 6 bis 11 durchgeführt wird.

12. Verfahren nach einem der Ansprüche 1 bis 11,
**dadurch gekennzeichnet,**
**dass** in den Schritten 1 und/oder 2 gereinigte und lyophilisierte Enzyme eingesetzt werden.

13. Verfahren nach einem der Ansprüche 1 bis 12,
**dadurch gekennzeichnet,**
**dass** die Schritte 1 und/oder 2 *in vivo* vorgenommen werden.

14. Verfahren nach Anspruch 13,
**dadurch gekennzeichnet,**
**dass** als Produktionsstamm *E. coli* eingesetzt wird.

15. Verfahren nach Anspruch 13 oder 14,
**dadurch gekennzeichnet,**
**dass** die Gene kodierend für die Lyase und/oder Transaminase in einen Vektor ligiert sind.

## Claims

1. Method for producing (1S,2S)-norpseudoephedrine,
**characterised in that**
benzaldehyde is in a first reaction step with an acetyl donor according to formula (1) 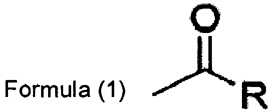 with R = H or COOH by means of the (S)-selective lyase ApPDC-E469G from *Acetobacter pasteurianus* is converted into an enantiomer mixture according to formulas (2) and (3) 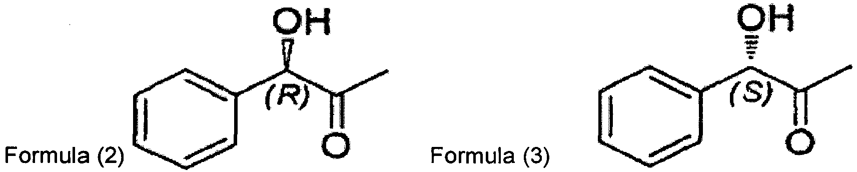 and the compound according to formula (3) is converted into (1S,2S)-norpseudoephedrine in a second step with an amine donor by means of an (S)-selective transaminase.

2. Method according to claim 1,
**characterised in that**
a magnesium salt and thiamine diphosphate is used as a cofactor.

3. Method according to one of claims 1 or 2,
**characterised in that**
step 1 is carried out at a pH value of 5 to 9.

4. Method according to one of claims 1 to 3,
**characterised in that**, the compound according to formula (2) 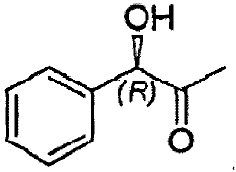 as the product of the first reaction step is converted into benzaldehyde and acetaldehyde by means of a benzaldehyde lyase.

5. Method according to one of claims 1 to 4,
**characterised in that**,
the acetyl donor is added in excess.

6. Method according to one of claims 1 to 5, **characterised in that**, the intermediate product according to formula (3) 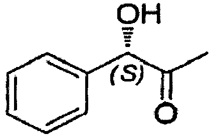 is separated.

7. Method according to one of claims 1 to 6, **characterised in that** the compound according to formula (3) 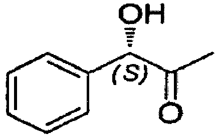 is converted in a second step with an *(S)-selective transaminase from a Chromobacterium, Alcaligens denitrificans, Arthrobacter citreus, Bacillus megaterium, Pseudomonas fluoreszens, Vibrio flurialis or Caulobacter crescentus.*

8. Method according to claim 7,
**characterised in that** CV2025 from *Chromobacterium violacaeum* is used as the (S)-selective transaminase.

9. Method according to one of claims 1 to 8,
**characterised in that**
a component from the group
methylbenzylamine, benzylamine,
isopropylamine, L-alanine, (±)-1-methyl-
3phenylpropylamine or (±)-1-aminoindane is
used as the amine donor in the second step.

10. Method according to one of claims 1 to 9,
**characterised in that**
pyridoxal-5'-phosphate is used as the cofactor in step 2.

11. Method according to one of claims 1 to 10,
**characterised in that**
step 2 is carried out at a pH value of 6 to 11.

12. Method according to one of claims 1 to 11,
**characterised in that**
purified and lyophilised enzymes are used in steps 1 and/or 2.

13. Method according to one of claims 1 to 12,
**characterised in that**
steps 1 and/or 2 are carried out *in vivo.*

14. Method according to claim 13,
**characterised in that**
*E. coli* is used as the production strain.

15. Method according to claim 13 or 14,
**characterised in that**
genes coding for the lyase and/or transaminase are ligated into a vector.

## Revendications

1. Procédé servant à fabriquer de la (1S,2S)-norpseudoéphédrine,
**caractérisé en ce**
**qu'**on fait réagir, lors d'une première étape de réaction, le benzaldéhyde avec un donneur acétyle de formule (1) 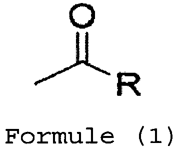 où R = H ou COOH, au moyen de la lyase (S)-sélective ApPDC-E469G issue de *Acetobacter pasteurianus* pour obtenir un mélange d'énantiomères des formules (2) et (3) 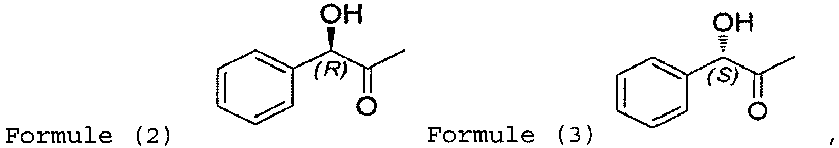 et en ce qu'on fait réagir , lors d'une deuxième étape, le composé de formule (3) avec un donneur d'amine, au moyen d'une transaminase (S)-sélective pour obtenir la (1S,2S)-norpseudoéphédrine.

2. Procédé selon la revendication 1,
**caractérisé en ce**
**qu'**un sel de magnésium ainsi qu'un disphosphate de thiamine sont employés en tant que cofacteurs.

3. Procédé selon l'une quelconque des revendications 1 ou 2,
**caractérisé en ce**
**que** l'étape 1 est effectuée à une valeur de pH allant de 5 à 9.

4. Procédé selon l'une quelconque des revendications 1 à 3,
**caractérisé**
**en ce qu'**on fait réagir le composé de formule (2) 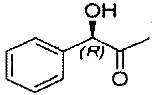 en tant que produit de la première étape de réaction, au moyen d'une lyase de benzaldéhyde dans du benzaldéhyde et dans de l'acétaldéhyde.

5. Procédé selon l'une quelconque des revendications 1 à 4,
**caractérisé en ce**
**que** le donneur d'acétyle est ajouté en excédent.

6. Procédé selon l'une quelconque des revendications 1 à 5,
**caractérisé en ce**
**que** le produit intermédiaire de formule (3) 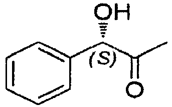 est séparé.

7. Procédé selon l'une quelconque des revendications 1 à 6,
**caractérisé en ce**
**qu'**on fait réagir le composé de formule (3) 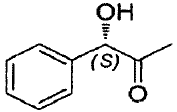 lors de la deuxième étape avec une transaminase (S)-sélectrive issue d'un *Chromobakterium, Alcaligens denitrificans, Arthrobacter citreus, Bacillus megaterium, Pseudomonas fluoreszens, Vibrio flurialis* ou *Caulobacter crescentus.*

8. Procédé selon la revendication 7,
**caractérisé en ce que** CV2025 issue de *Chromobakterium violacaeum* est employée en tant que transaminase (*S*)-sélective.

9. Procédé selon l'une quelconque des revendications 1 à 8,
**caractérisé en ce**
**qu'**un composant issu du groupe comprenant le méthylbenzylamine, le benzylamine, l'isopropylamine, la L-alanine, le (±)-1-méthyl-3phénylpropylamine ou le (±)-1-aminoindane est employé lors de la deuxième étape en tant que donneur d'amine.

10. Procédé selon l'une quelconque des revendications 1 à 9,
**caractérisé en ce**
**qu'**à l'étape 2, du pyridoxal-5'-phosphate est employé en tant que cofacteur.

11. Procédé selon l'une quelconque des revendications 1 à 10,
**caractérisé en ce**
**que** l'étape 2 est effectuée à une valeur de pH allant de 6 à 11.

12. Procédé selon l'une quelconque des revendications 1 à 11,
**caractérisé en ce**
**que** lors des étapes 1 et/ou 2, des enzymes nettoyées et lyophilisées sont employées.

13. Procédé selon l'une quelconque des revendications 1 à 12,
**caractérisé en ce**
**que** les étapes 1 et/ou 2 sont réalisées in vivo.

14. Procédé selon la revendication 13,
**caractérisé en ce**
**que** *E. coli* est employée en tant que souche de production.

15. Procédé selon la revendication 13 ou 14,
**caractérisé en ce**
**que** les gènes sont soumis à une ligation dans un vecteur en codant la lyase et/ou la transaminase.
